# FASCICULE DE BREVET EUROPEEN

(11) **EP 1 663 266 B1**
(45) Date de publication et mention de la délivrance du brevet: **20.02.2013**
(21) Numéro de dépôt: 04763951.3
(22) Date de dépôt: 10.08.2004
(51) Int. Cl.: A61K 36/064, A61K 31/716, A61K 31/736, A61P 3/10

(54) **ECORCES DE LEVURES POUR LE TRAITEMENT OU LA PREVENTION DE L'HYPERGLYCEMIE OU POUR LA STABILISATION DE LA GLYCEMIE**
HEFEZELLWÄNDE ZUR BEHANDLUNG ODER VORBEUGUNG DER HYPERGLYKÄMIE UND ZUR BLUTZUCKERSTABILISIERUNG
YEAST CELL WALLS FOR THE TREATMENT OR PREVENTION OF HYPERGLYCEMIA OR FOR THE STABILISATION OF GLYCEMIA

(30) Priorité: 11.08.2003 EP 03018222; 08.09.2003 EP 03020253
(43) Date de publication de la demande: 07.06.2006
(73) Titulaire: Lesaffre et Compagnie, 75001 Paris (FR)
(72) Inventeur: LESAFFRE, Lucien, F-59700 Marcq-en-Baroeul (FR)
(74) Mandataire: Cabinet Plasseraud
(86) Numéro de dépôt international: PCT/EP2004/008933
(87) Numéro de publication internationale: WO 2005/021015

(56) Documents cités:
- EP-A- 1 094 117
- DE-A- 4 413 023
- DE-A- 19 835 767
- FR-A- 2 825 004

## Description

La présente invention concerne le traitement et la prévention de l'hyperglycémie, ainsi que la stabilisation de la glycémie.

Un grand nombre de composés ont déjà été proposés pour la prévention de l'hyperglycémie, pour le traitement de l'hyperglycémie et/ou pour la stabilisation de la glycémie, en particulier en cas de diabète.

On a ainsi proposé des produits à base de levure, comme notamment de la levure en tant que telle, de l'extrait de levure, un facteur de tolérance au glucose à base de chrome (GTF) extrait d'une levure enrichie en chrome, et aussi une préparation cellulaire de levure de brasserie.

Ainsi, JP-A-61-167622 propose un agent de lutte contre le diabète à base d'une fraction cellulaire de levure de brasserie appelée paroi cellulaire dans ce document et obtenue par hydrolyse de levure de brasserie désamérisée pendant au moins 2 heures à une température de 50 à 70°C et extraction aqueuse des constituants solubles dans l'eau. Ladite fraction cellulaire de levure de brasserie présente notamment une teneur en glucanes d'environ 14,8%, une teneur en mannanes d'environ 13,9%. Ladite fraction cellulaire présente également une teneur en glycogène d'environ 24,9%. Le glycogène ou amidon de foie (en anglais : « liver starch » ; en allemand : « Leberstärke ») est une polysaccharide de réserve également présent dans les muscles et en particulier dans le foie. Ce glycogène est également une substance de réserve de la levure, utilisée par celle-ci comme source d'énergie pour assurer sa survie. Alors qu'il est un des composants principaux de la fraction cellulaire décrite dans cette demande japonaise, il ne fait pas partie de la paroi cellulaire des levures.

Selon la présente invention, il a maintenant été constaté qu'il est possible d'obtenir des écorces de levure efficaces en tant qu'agent pour la prévention et le traitement de l'hyperglycémie et présentant une teneur en glycogène faible, lesdites écorces de levure pouvant être obtenues par un procédé d'autolyse ou d'hydrolyse enzymatique simple.

Le terme « écorce de levure » se réfère à la fraction insoluble des cellules de levure, non enrichies en chrome, obtenue après une autolyse ou une hydrolyse enzymatique, essentiellement par des protéases, conduisant à la solubilisation d'au moins 50% et de préférence d'au moins 60% en masse des matières sèches des cellules de levure entières et conservant les polysaccharides de structure de la paroi cellulaire, c'est-à-dire les ß-glucanes et les mannanes, ces mannanes étant sous forme de mannoprotéines.

Cette autolyse ou hydrolyse enzymatique est conduite de manière à solubiliser l'essentiel des sucres de réserve de la cellule de levure que sont le glycogène et la tréhalose. Les écorces de levure sont obtenues par séparation de la fraction solubilisée par l'autolyse ou l'hydrolyse enzymatique, celle-ci ayant une durée de préférence d'au moins 18 heures. Les procédés préférés d'autolyse des crèmes de levure sont décrits pages 370 à 377 dans l'ouvrage de référence « Yeast Technology », 2ème édition, 1991, G. Reed et T.W. Nogodawithana, publié par Van Nostrand Reinhold, New York, ISBN 0-442-31892-8. Les écorces de levure ainsi obtenues sont ensuite typiquement séchées par un procédé classique de séchage, comme le séchage par atomisation ou le séchage sur rouleaux chauffés.

Les écorces de levure suivant l'invention sont des écorces de levures du genre *Saccharomyces* appartenant de préférence à l'espèce *Saccharomyces cerevisiae.*

Lesdites levures sont de préférence des levures de boulangerie. Une levure de boulangerie est une levure appartenant à l'espèce *Saccharomyces* cerevisiae, fabriquée à l'aide essentiellement d'une multiplication ou culture aérobie comme enseignée dans l'ouvrage de référence « Yeast Technology » cité ci-dessus et n'ayant servi avant son autolyse ou hydrolyse enzymatique à aucun usage, contrairement, par exemple, à de la levure de brasserie qui est un sous-produit de la fabrication de la bière et qui a donc servi à fabriquer de la bière avant sa récupération pour son autolyse ou hydrolyse enzymatique. Cette levure de brasserie a été multipliée essentiellement en anaérobie (la fabrication de la bière étant un processus anaérobie).

Les écorces de levure suivant l'invention présentent une teneur totale en glucanes et mannanes de la paroi cellulaire ( systématiquement exprimée en masse équivalente de respectivement glucose et mannose - voir méthodes de mesure ci-après ) d'au moins 34,0% en masse sur matières sèches, ainsi qu'une teneur en glycogène ( systématiquement exprimée en masse équivalente de glucose - voir méthodes de mesure ci-après ) inférieure à 10,0% en masse sur matières sèches

De préférence, les écorces de levure suivant l'invention présentent une teneur totale en glucanes et mannanes d'au moins 40,0% en masse sur matières sèches, encore de préférence d'au moins 45,0% en masse sur matières sèches

Egalement, de préférence, les écorces de levure suivant l'invention présentent une teneur en glycogène inférieure à 8,0% en masse sur matières sèches, encore de préférence inférieure à 5,0 % en masse sur matières sèches et plus de préférence encore inférieure à 3,0% en masse sur matières sèches.

De manière utile, les écorces de levure suivant l'invention présentent une teneur en protéines de 17,0 à 35,0% en masse sur matières sèches, de préférence de 18,0 à 26,0% en masse sur matières sèches

De manière intéressante, les écorces de levure suivant l'invention présentent une teneur en matières sèches d'au moins 90% en masse, de préférence, d'au moins 94% en masse et encore de préférence d'au moins 96 % en masse.

En général, la teneur totale en glucanes et mannanes de l'écorce de levure selon l'invention est inférieure ou égale à 70% en masse sur matières sèches. Elle peut en particulier être inférieure ou égale à 65% en masse sur matières sèches. Elle peut aussi être inférieure ou égale à 55% en masse sur matières sèches.

Selon une variante de l'invention, afin d'éliminer totalement ou quasi-totalement le glycogène de l'écorce de levure, des écorces de levure suivant l'invention obtenues comme décrit ci-dessus sont mises en suspension aqueuse, chauffées en milieu alcalin entre 70°C et 100°C pendant trois heures maximum, et la fraction solubilisée par ce traitement est éliminée, la fraction non-solubilisée restante étant récupérée et généralement séchée. Par exemple, une suspension à environ 12% de matières sèches d'écorces de levure en milieu aqueux alcalin sodé peut être chauffée à 85°C pendant deux heures. La fraction solubilisée contenant tout le glycogène, mais aussi une grande partie, voire la totalité, des mannoprotéines est éliminée par centrifugation et lavage.

Un tel traitement permet de réaliser des écorces de levure suivant l'invention ayant une teneur totale en glucanes et mannanes de 55% à 70% en masse sur matières sèches, de préférence de 60% à 70% en masse et encore de préférence de 60 à 65% en masse sur matières sèches. Un tel traitement permet notamment de réaliser des écorces de levure qui contiennent moins de 1,0% en masse de glycogène sur matières sèches, de préférence moins de 0,1% en masse de glycogène sur matières sèches. Ces écorces de levure peuvent ne pas contenir de mannanes.

Ces écorces de levure à teneur totale élevée en glucanes et mannanes correspondent à une forme d'exécution d'un agent suivant l'invention pour le traitement ou la prévention de l'hyperglycémie ou pour la stabilisation de la glycémie.

Elles sont appropriées pour les préparations suivant l'invention pour le traitement ou la prévention de l'hyperglycémie ou pour la stabilisation de la glycémie. Elles peuvent, suivant l'invention, être utilisées dans la préparation d'une composition thérapeutique pour le traitement ou la prévention de l'hyperglycémie ou pour la stabilisation de la glycémie.

### Méthode pour la mesure de la teneur en glycogène

On ajoute à un échantillon de 20 mg d'écorces de levure sèches, c'est-à-dire à une teneur en matières sèches d'au moins 90% en masse, 0,5ml de Na₂CO₃ 0,25M et on maintient ce mélange à 95°C pendant 4 heures.

Le mélange est ensuite porté à un pH de 5,2 par addition de 0,3ml d'acide acétique 1M et de 1,2ml d'acétate de sodium 0,2M et en mélangeant les ingrédients. On ajoute de l'eau distillée jusqu'à un volume total de 2ml.

0,5 ml de la suspension ainsi obtenue sont incubés pendant 15 heures en présence d'un excès d'amyloglucosidase *d'Aspergillus niger,* telle que commercialisée par la société ROCHE sous le numéro Cat. No. 102 857, à 55°C.

Après centrifugation, on dose le glucose libéré par dosage enzymatique.

Le dosage enzymatique de glucose est décrit notamment dans le manuel « Methods of Biochemical Analysis and Food Analysis - using Single Réagents », publié par BOEHRINGER MANNHEIM GmbH Biochemica, © 1989, pages 50 à 55, et est de préférence réalisé en utilisant le « Test-Combination D-Glucose/-Fructose », Cat. No. 139 106 de la filiale de la société ROCHE : BOEHRINGER MANNHEIM GmbH / R-BIOPHARM GmbH à Darmstadt, Allemagne.

La quantité (en mg) de glucose ainsi dosée correspond à la quantité de glycogène présente dans l'échantillon exprimée en masse équivalente de glucose.

### Méthode pour la mesure de la teneur totale en glucanes et mannanes

On soumet un échantillon de 20 mg d'écorces de levure sèches, c'est-à-dire à une teneur en matières sèches d'au moins 90% en masse, à une hydrolyse acide en le mélangeant avec 20 ml de HCl 2N, et on maintient le mélange en tube à vis fermé pendant 4 heures à 103°C en étuve avec agitation toutes les 15mn.

Ensuite, on neutralise la solution acide ainsi obtenue et on dose par voie enzymatique la quantité de glucose et respectivement de mannose dans la solution neutralisée.

Ce dosage enzymatique de glucose et mannose est également décrit aux pages 50 à 55 du manuel cité ci-dessus et est de préférence réalisé en utilisant le « Test-Combination » Cat. No. 139 106.

On fait la différence entre d'une part la quantité de glucose (exprimée en mg) dosée suivant cette méthode et la quantité de glucose (également exprimée en mg) dosée pour ces écorces de levure par la méthode pour la mesure de la teneur en glycogène ci-dessus.

Cette différence (en mg) entre les deux quantités de glucose dosées correspond à la quantité totale de glucanes présente dans l'échantillon, cette quantité étant exprimé en masse équivalente de glucose.

La quantité (en mg) de mannose dosée correspond à la quantité totale de mannanes présente dans l'échantillon, cette quantité étant exprimée en masse équivalente de mannose.

La présente invention concerne en premier lieu un agent pour utilisation dans le traitement de l'hyperglycémie constitué d'écorces de levure telles que définies ci-dessus, c'est-à-dire les écorces de levure suivant l'invention. Le traitement de l'hyperglycémie concerne principalement la réduction de la glycémie, c'est à dire du taux de glucose sanguin.

Cet agent suivant l'invention peut être utile dans plusieurs cas d'hyperglycémie, comme notamment les cas énumérés ci-après :
(a) pour le traitement de l'hyperglycémie en cas de diabète de type 2 (le diabète de type 2 étant appelé ci-après « condition (a) ») ;
(b) pour le traitement de l'hyperglycémie en cas de diabète de gestation ou grossesse (le diabète de gestation ou grossesse étant appelé ci-après « condition (b) ») ;
(c) pour le traitement de l'hyperglycémie en cas de pré-diabète (le pré-diabète étant appelé ci-après « condition (c) ») ;
(d) pour le traitement de l'hyperglycémie post-prandiale (l'état post-prandial étant appelé ci-après « condition (d) »).

La présente invention concerne également un agent pour utilisation dans la prévention de l'hyperglycémie, ledit agent étant constitué d'écorces de levure suivant l'invention. La prévention de l'hyperglycémie concerne principalement le maintien de la glycémie à des niveaux inférieurs à l'hyperglycémie.

Ledit agent peut, en particulier, être un agent pour utilisation dans la prévention de l'hyperglycémie en cas d'au moins une des conditions (a), (b), (c) ou (d) telles que définies ci-dessus.

La présente invention concerne aussi un agent pour utilisation dans la stabilisation de la glycémie, ledit agent étant constitué d'écorces de levure suivant l'invention. La stabilisation de la glycémie concerne principalement le maintien de la glycémie à des niveaux inférieurs à l'hyperglycémie et supérieurs à l'hypoglycémie.

Ledit agent peut, en particulier, être un agent pour utilisation dans la stabilisation de la glycémie en cas d'au moins une des conditions (a), (b), (c) ou (d).

L'agent suivant l'invention, dans ses différentes formes de mise en oeuvre, peut être administré sous différentes formes ou présentations, seul ou en combinaison avec d'autres ingrédients, comme, par exemple, un ou plusieurs autres ingrédients thérapeutiquement actifs et/ou un ou plusieurs excipients.

Ainsi, la présente invention concerne une préparation pour utilisation dans le traitement de l'hyperglycémie qui comprend des écorces de levure suivant l'invention, telles que définies ci-dessus. La dite préparation comprend en d'autres mots un agent suivant l'invention.

Ladite préparation suivant l'invention peut, en particulier, être une préparation pour utilisation dans le traitement de l'hyperglycémie en cas d'au moins une des condition (a), (b), (c) ou (d).

La présente invention concerne également une préparation pour utilisation dans la prévention de l'hyperglycémie qui comprend des écorces de levure suivant l'invention.

Ladite préparation peut, en particulier, être une préparation pour utilisation dans la prévention de l'hyperglycémie en cas d'au moins une des conditions (a), (b), (c) ou (d).

La présente invention concerne aussi une préparation pour utilisation dans la stabilisation de la glycémie qui comprend des écorces de levure suivant l'invention.

Ladite préparation peut, en particulier, être une préparation pour utilisation dans la stabilisation de la glycémie en cas d'au moins une des conditions (a), (b), (c) ou (d).

La préparation suivant l'une quelconque des formes de réalisation ci-dessus est généralement une préparation pour administration par voie orale.

La préparation peut notamment se présenter sous forme de comprimé, de capsule, de pilule, de poudre, de granulés ou de suspension.

La préparation suivant l'invention peut également comprendre un ou plusieurs agents thérapeutiquement actifs, et notamment un ou plusieurs agents hypoglycémiants. La préparation peut ainsi comprendre une ou plusieurs vitamines, un ou plusieurs minéraux alimentaires, etc.

La préparation peut également comprendre un ou plusieurs excipients pharmaceutiquement acceptables.

La préparation suivant l'invention peut en particulier se présenter sous forme d'une dose d'ingestion correspondant à une quantité de matières sèches d'écorces de levure suivant l'invention inférieure à 10 g, de préférence de 1 à 8 g, encore de préférence de 1 à 7 g.

La présente invention concerne également l'utilisation dans la préparation d'une composition thérapeutique ou médicament des écorces de levure suivant l'invention, dont différentes formes de réalisation sont définies ci-dessus.

L'invention concerne notamment l'utilisation de ces écorces de levure dans la production d'une des compositions suivant l'invention telle que définie ci-dessus.

La présente invention concerne ainsi l'utilisation d'écorces de levure suivant l'invention dans la préparation d'une composition thérapeutique pour le traitement de l'hyperglycémie.

Les écorces de levure suivant l'invention peuvent en particulier être utilisées dans la préparation d'une composition thérapeutique pour le traitement de l'hyperglycémie en cas d'au moins une des conditions (a), (b), (c) ou (d).

La présente invention concerne également l'utilisation d'écorces de levure suivant l'invention dans la préparation d'une composition thérapeutique pour la prévention de l'hyperglycémie.

Les écorces de levure suivant l'invention peuvent en particulier être utilisées dans la préparation d'une composition thérapeutique pour la prévention de l'hyperglycémie en cas d'au moins une des conditions (a), (b), (c) ou (d).

La présente invention concerne aussi l'utilisation d'écorces de levure suivant l'invention dans la préparation d'une composition thérapeutique pour la stabilisation de la glycémie.

Les écorces de levure suivant l'invention peuvent en particulier être utilisées dans la préparation d'une composition thérapeutique pour la stabilisation de la glycémie en cas d'au moins une des conditions (a), (b), (c) ou (d).

La composition pharmaceutique est en règle générale une composition pour administration par voie orale.

La composition pharmaceutique peut notamment se présenter sous forme de comprimé, de capsule, de pilule, de poudre, de granulés ou de suspension.

Les écorces de levure suivant l'invention peuvent notamment être utilisées dans la préparation d'une composition pharmaceutique comprenant également :
- un ou plusieurs agents thérapeutiquement actifs, et notamment un ou plusieurs agents hypoglycémiants, et/ou
- une ou plusieurs vitamines, un ou plusieurs minéraux alimentaires, etc.

La composition pharmaceutique peut également comprendre un ou plusieurs excipients pharmaceutiquement acceptables.

Les écorces de levure suivant l'invention peuvent en particulier être utilisées pour la préparation d'une composition thérapeutique sous forme d'une dose d'ingestion correspondant à une quantité de matières sèches d'écorces de levure suivant l'invention inférieure à 10 g, de préférence de 1 à 8 g, encore de préférence de 1 à 7 g.

### Exemples

### A. Méthode d'obtention d'écorces de levure suivant l'invention

Une crème aqueuse (c'est-à-dire une suspension de cellules de levure dans l'eau) de *Sacharomyces cerevisiae,* ayant une teneur en matières sèches entre 12 et 18% en masse est soumise à une hydrolyse à l'aide des enzymes endogènes desdites cellules de levure, éventuellement avec ajout de protéases exogènes aux cellules de levure, telle que par exemple la papaïne. L'hydrolyse est effectuée à 50°C pendant 24 heures, de manière à solubiliser au moins 60% en masse des matières sèches des cellules de levure.

De manière pratique, en général, les autolyses ou hydrolyses enzymatiques selon l'invention sont réalisées entre 45°C et 55°C pendant 18 à 36 heures, sans l'emploi d'aucun enzyme pouvant solubiliser les glucanes ou les mannoprotéines.

On sépare la fraction solubilisée de la fraction insoluble par plusieurs étapes successives de centrifugation et de lavage avec de l'eau.

La fraction insoluble est séchée sur des tambours chauffés jusqu'à une teneur en matières sèches de 95% en masse. Les agglomérats formés sont enlevés par tamisage.

Les écorces de levure ainsi obtenues présentent :
- une teneur en matières sèches de 95% en masse,
- une teneur en protéines Nx6,25 de 20,2% en masse sur matières sèches
- une teneur totale en glucanes et mannanes de 43% sur matières sèches
- une teneur en glycogène de 8% en masse sur matières sèches.

### B. Prévention et traitement de l'hyperglycémie au moyen d'une préparation suivant l'invention

Une patiente invalide de 82 ans était diagnostiquée comme souffrante de diabète de type 2.

Ne pouvant pas participer à des exercices physiques exigeants, le traitement initial était limité à un régime à basse teneur en matières grasses et sucres digestibles.

L'effet réalisé grâce à ce régime était limité. On observait en particulier que la glycémie de la patiente restait à une teneur en glucose sanguine de 10,0 à 12,0 mmol/litre, sachant qu'une teneur en glucose sanguine entre 4,0 et 7,0 mmol/litre, de préférence entre 4,2 et 6,0 mmol/litre, était recherchée.

Par la suite, le régime susmentionné a été complémenté par l'ajout de 3 à 4 g d'écorces de levure telles que décrites sous le point A ci-dessus, aux céréales du petit déjeuner.

12 Heures après la première ingestion desdites écorces de levure, la glycémie de la patiente était descendue à 6 mmol de glucose sanguin /litre.

En continuant l'ingestion de 3 à 4 g d'écorces de levure par jour lors du petit déjeuner, la glycémie de la patiente a été maintenue pendant au moins 4 mois entre 4,5 et 6 mmol de glucose sanguin /litre.

Aucune hyperglycémie post-prandiale ne s'est produite pendant ces 4 mois, même après des infractions au régime alimentaire prescrit, comme la consommation de chocolat.

Aucun effet secondaire négatif n'a été observé.

## Revendications

1. Agent pour utilisation dans le traitement de l'hyperglycémie, l'agent étant constitué d'écorces de levure du genre *Saccharomyces cerevisiae,* lesdites écorces de levure ayant :
• une teneur totale de glucanes et mannanes d'au moins 34,0% en masse sur matières sèches, de préférence d'au moins 40,0% en masse sur matières sèches et encore de préférence d'au moins 45,0% en masse sur matières sèches, et
• une teneur en glycogène inférieure à 10,0% en masse sur matières sèches, de préférence inférieure à 8,0% en masse sur matières sèches, encore de préférence inférieure à 5,0% en masse sur matières sèches, et plus de préférence encore inférieure à 3,0% en masse sur matières sèches.

2. Agent pour utilisation selon la revendication 1, dans lequel les écorces de levure ont une teneur totale en glucanes et mannanes inférieure ou égale à 70% en masse sur matières sèches.

3. Agent pour utilisation selon l'une quelconque des revendications 1 et 2, dans lequel les écorces de levure ont une teneur totale en glucanes et mannanes de 55% à 70% en masse sur matières sèches, de préférence de 60% à 70% en masse, et encore de préférence de 60% à 65% en masse sur matières sèches.

4. Agent pour utilisation selon l'une quelconque des revendications précédentes, dans lequel les écorces de levure ont une teneur en glycogène inférieure à 1,0% en masse sur matières sèches, de préférence inférieure à 0,1% en masse sur matières sèches.

5. Agent pour utilisation selon l'une quelconque des revendications précédentes, lequel les écorces de levure présentent une teneur en matières sèches supérieure ou égale à 90%, de préférénce supérieure ou égale à 94% et encore de préférence supérieure ou égalé à 96% en masse.

6. Agent pour l'utilisation dans la prévention de l'hyperglycémie, l'agent étant constitué d'écorces de levure telles que definies dans l'une quelconque des revendications précédentes.

7. Agent pour utilisation selon l'une quelconque des revendications précédentes, **caractérisé en ce que** l'hyperglycémie est une hyperglycémie en cas de diabète de type 2.

8. Agent pour utilisation dans la stabilisation de la glycémie, l'agent étant constitué d'écorces de levure telles que définies dans l'une quelconque des revendications 1 à 5.

9. Agent pour utilisation selon la revendication 8 pour la stabilisation de la glycémie en cas de diabète de type 2.

10. Préparation pour utilisation dans le traitement de l'hyperglycémie, préparation comprenant des écorces de levure telles que définies dans l'une quelconque des revendications 1 à 5.

11. Préparation pour utilisation dans la prévention de l'hyperglycémie, préparation comprenant des écorces de levure telles que définies dans l'une quelconque des revendications 1 à 5.

12. Préparation pour utilisation selon l'une quelconque des revendications 10 et 11, **caractérisée en ce que** l'hyperglycémie est une hyperglycémie en cas de diabète de type 2.

13. Préparation pour utilisation dans la stabilisation de la glycémie, préparation comprenant des écorces de levure telles que définies dans l'une quelconque des revendications 1 à 5.

14. Préparation pour utilisation selon la revendication 13 pour la stabilisation de la glycémie en cas de diabète de type 2.

## Claims

1. Agent for use in the treatment of hyperglycaemia, the agent being composed of yeast cell walls of the *Saccharomyces cerevisiae* type, the said yeast cell walls having :
• a total content of glucanes and mannanes of at least 34.0% by mass of dry materials, preferably at least 40.0% by mass of dry materials and even more preferably at least 45.0% by mass of dry materials, and
• a content of glycogen of less than 10.0% by mass of dry materials, preferably less than 8.0% by mass of dry materials, even more preferably of less than 5.0% of dry materials, and most preferably even less than 3.0% by mass of dry materials.

2. Agent for use according to claim 1 in which the yeast cell walls have a total content of glucanes and mannanes of less than or equal to 70% by mass of dry materials.

3. Agent for use according to any of claims 1 and 2 in which the yeast cell walls have a total content of glucanes and mannanes of 55% to 70% by mass of dry materials, preferably of 60 % to 70% by mass, and even more preferably of 60% to 65% by mass of dry materials.

4. Agent for use according to any of the preceding claims, in which the yeast cell walls have a content of glycogen of less than 1.0% by mass of dry materials, preferably less than 0.1 % by mass of dry materials.

5. Agent for use according to any of the preceding claims, in which the yeast cell walls have a content of dry materials higher than or equal to 90%, preferably higher than or equal to 94% and even more preferably higher than or equal to 96% by mass.

6. Agent for use in the prevention of hyperglycaemia, the agent being comprised of yeast cell walls as defined in any of the preceding claims.

7. Agent for use according to any of the preceding claims, **characterised in that** the hyperglycaemia is hyperglycaemia in the case of type 2 diabetes.

8. Agent for use in the stabilisation of glycaemia, the agent being composed of yeast cell walls as defined in any of claims 1 to 5.

9. Agent for use according to claim 8 for the stabilisation of glycaemia in the case of type 2 diabetes.

10. Preparation for use in the treatment of hyperglycaemia, preparation comprising yeast cell walls as defined in any of claims 1 to 5.

11. Preparation for use in the prevention of hyperglycaemia, preparation comprising yeast cell walls as defined in any of claims 1 to 5.

12. Preparation for use according to any of claims 10 and 11 **characterised in that** the hyperglycaemia is a hyperglycaemia in the case of type 2 diabetes.

13. Preparation for use in the stabilisation of glycaemia, preparation comprising yeast cells walls as defined in any of claims 1 to 5.

14. Preparation for use according to claim 13 for the stabilisation of glycaemia in the case of type 2 diabetes.

## Patentansprüche

1. Mittel zur Verwendung bei der Behandlung von Hyperglykämie, wobei das Mittel aus Zellwänden von Hefe der Gattung *Saccharomyces cerevisiae* gebildet ist, wobei die Hefezellwände aufweisen:
- einen Gesamtgehalt an Glucanen und Mannanen von mindestens 34,0 Massen-% bezogen auf das Trockenmaterial, bevorzugt von mindestens 40,0 Massen-% bezogen auf das Trockenmaterial und mehr bevorzugt von mindestens 45,0 Massen-% bezogen auf das Trockenmaterial, und
- einen Gehalt an Glykogen von kleiner als 10,0 Massen-% bezogen auf das Trockenmaterial, bevorzugt von kleiner als 8,0 Massen-% bezogen auf das Trockenmaterial, noch bevorzugt von kleiner als 5,0 Massen-% bezogen auf das Trockenmaterial, und noch mehr bevorzugt von kleiner als 3,0 Massen-% bezogen auf das Trockenmaterial.

2. Mittel zur Verwendung gemäß Anspruch 1, wobei die Hefezellwände einen Gesamtgehalt an Glucanen und Mannanen von kleiner als oder gleich 70 Massen-% bezogen auf das Trockenmaterial haben.

3. Mittel zur Verwendung gemäß einem der Ansprüche 1 und 2, wobei die Hefezellwände einen Gesamtgehalt an Glucanen und Mannanen von 55 bis 70 Massen-% bezogen auf das Trockenmaterial, bevorzugt von 60 bis 70 Massen-%, und mehr bevorzugt von 60 bis 65 Massen-% bezogen auf das Trockenmaterial haben.

4. Mittel zur Verwendung gemäß einem der vorhergehenden Ansprüche, wobei die Hefezellwände einen Gehalt an Glykogen von kleiner als 1,0 Massen-% bezogen auf das Trockenmaterial, bevorzugt von kleiner als 0,1 1 Massen-% bezogen auf das Trockenmaterial haben.

5. Mittel zur Verwendung gemäß einem der vorhergehenden Ansprüche, wobei die Hefezellwände einen Gehalt an Trockenmaterial von größer als oder gleich 90%, bevorzugt von größer als oder gleich 94% und mehr bevorzugt von größer als oder gleich 96 Massen-% aufweisen.

6. Mittel zur Verwendung bei der Prävention von Hyperglykämie, wobei das Mittel aus Hefezellwänden, wie in einem der vorhergehenden Ansprüche definiert, gebildet ist.

7. Mittel zur Verwendung gemäß einem der vorhergehenden Ansprüche, **dadurch gekennzeichnet, dass** die Hyperglykämie eine Hyperglykämie im Fall von Diabetes Typ 2 ist.

8. Mittel zur Verwendung bei der Blutzuckerstabilisierung , wobei das Mittel aus Hefezellwänden, wie in einem der Ansprüche 1 bis 5 definiert, gebildet ist.

9. Mittel zur Verwendung gemäß Anspruch 8 zur Blutzuckerstabilisierung im Fall von Diabetes Typ 2.

10. Zubereitung zur Verwendung bei der Behandlung von Hyperglykämie, wobei die Zubereitung Hefezellwände, wie in einem der Ansprüche 1 bis 5 definiert, umfasst.

11. Zubereitung zur Verwendung bei der Prävention von Hyperglykämie, wobei die Zubereitung Hefezellwände, wie in einem der Ansprüche 1 bis 5 definiert, umfasst.

12. Zubereitung zur Verwendung gemäß einem der Ansprüche 10 und 11, **dadurch gekennzeichnet, dass** die Hyperglykämie eine Hyperglykämie im Fall von Diabetes Typ 2 ist.

13. Zubereitung zur Verwendung bei der Blutzuckerstabilisierung, wobei die Zubereitung Hefezellwände, wie in einem der Ansprüche 1 bis 5 definiert, umfasst.

14. Zubereitung zur Verwendung gemäß Anspruch 13 zur Blutzuckerstabilisierung im Fall von Diabetes Typ 2.
